# EUROPÄISCHE PATENTSCHRIFT

(11) **EP 4 028 107 B1**
(45) Veröffentlichungstag und Bekanntmachung des Hinweises auf die Patenterteilung: **08.07.2026**
(21) Anmeldenummer: 20767972.1
(22) Anmeldetag: 12.08.2020
(51) Int. Cl.: A61M 25/10

(54) **VERFAHREN ZUR REDUZIERUNG DES AUSSENDURCHMESSERS DES BALLONS EINES BALLONKATHETERS**
METHOD OF REDUCTION OF THE OUTER DIAMETER OF THE BALLOON OF A BALLOON CATHETER
MÉTHODE DE RÉDUCTION DU DIAMÈTRE EXTÉRIEUR DU BALLON D'UN CATHÉTER À BALLON

(30) Priorität: 09.09.2019 DE 102019124056; 30.04.2020 DE 102020111805
(43) Veröffentlichungstag der Anmeldung: 20.07.2022
(73) Patentinhaber: Ruebben, Alexander, 98000 Monaco (MC)
(72) Erfinder: Ruebben, Alexander, 98000 Monaco (MC)
(74) Vertreter: Schneiders & Behrendt Bochum
(86) Internationale Anmeldenummer: PCT/EP2020/072571
(87) Internationale Veröffentlichungsnummer: WO 2021/047848

(56) Entgegenhaltungen:
- WO-A1-2013/066566
- WO-A1-2016/050303
- JP-A- 2003 062 081
- JP-A- 2005 211 492

## Beschreibung

Die Erfindung betrifft ein Verfahren zur Reduzierung des Außendurchmessers des Ballons eines Ballonkatheters im deflatierten Zustand.

Der Einsatz von Ballonkathetern gehört heutzutage zum Standard im klinischen Alltag. Ihre Verwendung im Rahmen von intravaskulären Interventionen bezieht sich meist auf die Aufweitung verengter Gefäßstellen, entweder mit Hilfe des Ballonkatheters an sich oder in Kombination mit weiteren Medizinprodukten wie beispielsweise bei ballonexpandierbaren Stents.

Da sich die vorliegende Erfindung insbesondere mit Verfahren befasst, die den Ballon eines Ballonkatheters im nicht expandierten Zustand betreffen, ist nachfolgend grundsätzlich von einem nicht expandierten, also deflatierten Zustand des Ballons auszugehen, soweit nichts anderes ausdrücklich erwähnt wird. Zudem werden nachfolgend die Begriffe "Durchmesser des Ballonkatheters", "Durchmesser des Ballons" sowie "Außendurchmesser des Ballonkatheters" und "Außendurchmesser des Ballons" synonym verwendet und beziehen sich immer auf den Ballonanteil des Katheters im deflatierten Zustand, soweit nicht ausdrücklich etwas anderes erwähnt wird. Ebenso werden die Begriffe "Ballonkatheter" und "Katheter" im Sinne dieser Anmeldung synonym für einen Katheter mit einem Ballon verwendet, es sei denn, es wird speziell auf einen Katheter ohne Ballon Bezug genommen.

Zu den wesentlichen Kenngrößen, die bei der Auswahl eines für die jeweilige Intervention geeigneten Ballonkatheters zu berücksichtigen sind, gehören die Länge des Ballons und sein nomineller Außendurchmesser im expandierten Zustand. Diese beiden Kenngrößen des Ballons werden in der Regel in Relation zu der Beschaffenheit der Gefäßverengung gewählt. Neben weiteren Faktoren sind bei der Auswahl eines geeigneten Ballonkatheters auch der Innendurchmesser und die Morphologie des betroffenen Gefäßes zu berücksichtigen. Je gewundener ein Gefäß beispielsweise ist, desto flexibler muss auch der gewählte Ballonkatheter sein. Bei der Flexibilität spielt unter anderem auch der Außendurchmesser des Ballons im nicht expandierten Zustand eine Rolle. Da der Ballon in der Regel auch im nicht expandierten Zustand den Bereich des Ballonkatheters mit dem größten Außendurchmesser bildet, ist dies bei der Auswahl eines geeigneten Katheters ein weiterer limitierender Faktor. Nicht zuletzt muss sich der Ballonkatheter auch im nicht expandierten Zustand nicht nur einfach durch das Gefäßsystem zum Zielort, der Läsion, vorschieben lassen, sondern er muss auch an der Läsion platziert werden können.

Es existieren also verschiedene Faktoren, die die Auswahl eines geeigneten Ballonkatheters beeinflussen, darunter auch die Biegsamkeit des Katheters und insbesondere die Biegsamkeit im Bereich des Ballons.

Grundsätzlich ist für eine Intervention zur Gefäßweitung ein solcher Ballonkatheter zu bevorzugen, der einen möglichst geringen Außendurchmesser aufweist. Ein Katheter sollte zunächst möglichst flexibel sein, damit er auf dem Weg zum Zielgefäß auch durch verschlungene und/oder enge Gefäße geschoben werden kann. Ein wesentlicher Faktor für die Flexibilität eines Ballonkatheters ist neben den verwendeten Materialien der Außendurchmesser im Bereich des Ballons. So kann man vereinfacht sagen, dass sich ein Katheter umso leichter auch durch verschlungene und/oder enge Gefäße navigieren beziehungsweise vorschieben lässt, desto geringer sein Außendurchmesser auch im Bereich des Ballons ist.

Bei der Auswahl ist zudem auf die bereits angesprochene Biegsamkeit bzw. Flexibilität auch und gerade im Bereich des gefalteten Ballons zu achten. Dabei werden durch die Flexibilität sowohl die Navigierbarkeit des Katheters (*"navigation"*) als auch die für den Vorschub des Katheters benötigte Kraft *("pushability")* beeinflusst. Je biegsamer, sprich flexibler, ein Ballonkatheter ist, desto besser und einfacher lässt er sich selbst durch gewundene Gefäße schieben.

Der Ballonkatheter muss zudem, wie bereits erwähnt, vor der Expansion die zu behandelnde Gefäßverengung passieren können. Genauer gesagt muss der Ballon des Katheters in der Gefäßverengung platziert werden können, um diese dann bei Expansion aufzuweiten. Je stärker die Gefäßverengung ausgebildet ist, desto weiter ist das Gefäß an dieser Stelle verschlossen und desto kleiner ist folglich der noch offene und somit passierbare Bereich. Entsprechend kann eine solche Gefäßverengung besser oder überhaupt nur mit einem Ballon passiert werden, dessen Querschnitt kleiner oder gleich dem passierbaren Bereich der Gefäßverengung ist. Diese Anforderung erfüllen Katheter mit einem geringeren Außendurchmesser eher als solche mit einem größeren Außendurchmesser.

Ziel ist es also, Ballonkatheter mit einem möglichst kleinen Außendurchmesser zu versehen, damit diese für eine größtmögliche Anzahl von Interventionen in Frage kommen. Für die Reduzierung des Außendurchmessers eines Ballons im nicht expandierten Zustand sind verschiedene Techniken bekannt.

Typischerweise liegt der deflatierte Ballon eines fertigen Katheters in einem gefalteten Zustand vor. Je nach Größe des Ballons können dabei unterschiedlich viele Falten ausgebildet sein, die anschließend um die Achse des Katheters in gleicher Richtung aufgewunden werden. Hierdurch wird bereits eine erste deutliche Reduktion des Außendurchmessers erreicht.

Betrachtet man einen so gefalteten Ballon im Querschnitt, dann erkennt man immer noch Zwischenräume zwischen den einzelnen aufgewundenen Falten untereinander und zwischen Falten und dem Katheterschaft. Um auch diese Zwischenräume weiter zu reduzieren, werden im Stand der Technik verschiedene Methoden vorgeschlagen. Beispielhaft sei hier das sogenannte Cut-Back-Verfahren erwähnt, bei dem der gefaltete Ballon mit übergezogenem Schlauch durch eine in der Regel trichterartige Düse geführt wird. Der Schlauch wird hierdurch gestreckt und verringert seinen Durchmesser, wodurch sich folglich auch der Durchmesser des in dem Schlauch befindlichen Ballons verringert. Ein solches Verfahren wird in der WO 2016/050303 A1 beschrieben.

Die Reduzierung des Ballondurchmessers mit bekannten Methoden führt durch die mehrlagige und gefaltete Anordnung immer auch zu einer Versteifung des Ballons. Dies hat im Bereich des gefalteten Ballons eine verminderte Biegsamkeit zur Folge, was sich nachteilig auf die Manövrierbarkeit und den Vorschub des Katheters gerade in stark gewundenen Gefäßen auswirken kann. Zum Teil wird der Ballon bei Verfahren aus dem Stand der Technik auch stark mechanisch belastet.

Es ist daher Aufgabe der Erfindung, ein Verfahren zur Verfügung zu stellen, das eine Reduzierung des Außendurchmessers eines nicht expandierten Ballons eines Ballonkatheters ermöglicht, ohne dabei die Nachteile bekannter Verfahren aufzuweisen.

Gelöst wird diese Aufgabe durch eine Erfindung mit den Merkmalen des Anspruchs 1. Vorteilhafte Ausgestaltungen sind jeweils Gegenstand der abhängigen Ansprüche. Es ist darauf hinzuweisen, dass die in den Ansprüchen einzeln aufgeführten Merkmale auch in beliebiger und technologisch sinnvoller Weise miteinander kombiniert werden können und somit weitere Ausgestaltungen der Erfindung aufzeigen.

Im Sinne der Erfindung werden unter dem Begriff "Restriktionselement" sowohl bandartige längliche Objekte verstanden, die sich insbesondere zum spiralförmigen Umwickeln eines länglichen Ballons eignen, als auch Ringelemente, die sich zum Aufschieben auf einen länglichen Ballon eignen.

Zur Vereinfachung soll nachfolgend im Fall von band- bzw. schnurartigen Restriktionselementen allgemein von "Bändern" gesprochen werden, im Fall von ringförmigen Restriktionselementen allgemein von "Ringen".

Ein erfindungsgemäßes Verfahren umfasst zumindest die folgenden Schritte:
(A) Bereitstellen eines Ballonkatheters;
(B) Anbringen von zumindest einem Restriktionselement auf zumindest einem Teil des Ballons des Ballonkatheters, wobei das zumindest eine Restriktionselement als Band oder Ring vorgesehen ist;
(C) Durchführen des gemäß Schritt (B) präparierten Ballons durch eine Öffnung; und
(D) Aufbringen einer Umhüllung auf den Ballon.

Vorzugsweise wird der Ballon des Ballonkatheters vor dem Anbringen des Restriktionselements in Falten gelegt, im Prinzip kann das Verfahren jedoch auch mit einem nicht gefalteten Ballon durchgeführt werden. Eine erste Durchmessereduzierung durch Faltung ist jedoch sinnvoll, da die Durchmesserreduzierung durch das erfindungsgemäße Verfahren dann nur noch verstärkt werden muss und die Faltung zudem eine geordnete Inflation des Ballons erlaubt.

Die Anbringung des mindestens einen Restriktionselements auf zumindest einem Teil des Ballons kann erfindungsgemäß auf unterschiedliche Weise erfolgen.

Bei der Verwendung von Bändern als Restriktionselemente ist die Anbringung auf dem Ballon beispielsweise durch eine Anzahl spiralförmiger bzw. schraubenförmiger oder helikaler Umwicklungen - die nachfolgend der Einfachheit halber als spiralförmige Umwicklungen zusammengefasst werden sollen - vorgesehen.

Bei der Verwendung von Ringen als Restriktionselemente ist die Anbringung auf dem Ballon beispielsweise durch ein Aufschieben der Ringe auf den Ballon vorgesehen. In der Regel werden mehrere Ringe auf den Ballon aufgebracht, typischerweise 2 bis 10, beispielsweise 3 bis 6.

Eine Kombination von Ringen und Bändern ist denkbar.

Sofern eine spiralförmige Umwicklung vorgesehen ist, ist die Umwicklung mit nur einem Band bevorzugt. Vorzugsweise erfolgt die spiralförmige Umwicklung nur in einer Richtung, um dem Ballon eine entsprechende Spiralstruktur aufzuprägen. Diese Spiralstruktur bleibt nach dem Durchführen durch die Öffnung auch dann erhalten, wenn das Band oder die Bänder wieder entfernt werden. In der Regel ist eine einlagige spiralförmige Umwicklung des Ballons ausreichend, denkbar ist jedoch auch eine mehrlagige Umwicklung auch mit mehreren Bändern.

Die Umwicklung kann in der Weise erfolgen, dass zwischen jeder Windung des Bandes noch eine kleine Lücke vorliegt, die Umwicklung kann jedoch auch lückenlos vorgesehen sein. Bevorzugt ist die Umwicklung des Ballons in der Weise, dass eine Lücke vorliegt, die Windungen also einen gewissen Abstand voneinander haben. Dieser liegt vorteilhafterweise bei 0,5 bis 7 mm, weiter bevorzugt bei 1 bis 5 mm. Die Umwicklung des Ballons mit dem Band oder den Bändern sollte möglichst stramm erfolgen, entweder indem die Umwicklung selbst bereits stramm erfolgt, d. h. unter Ausübung einer in Längsrichtung wirkenden Zugkraft auf das jeweilige Band, oder indem nach dem Umwickeln das jeweilige Band strammgezogen wird, was insbesondere durch auf die Enden einwirkende Zugkräfte erreicht werden kann.

Bei der Verwendung von Bändern sind flache Bänder mit einer gewissen Breite von ≥ 2 mm bevorzugt. Besonders bevorzugt ist eine Breite von 0,5 bis 2 cm. Unter Breite wird in diesem Zusammenhang die Ausdehnung des Bandes parallel zur Oberfläche des Ballons und orthogonal zur Längsrichtung des Bandes verstanden. Die Länge des Bandes richtet sich nach der Länge und dem Durchmesser des zu präparierenden Ballons und nach der Art der Wicklung und wird entsprechend ausgewählt.

Sofern Ringe als Restriktionselemente vorgesehen sind, so ist die Anbringung mehrerer Ringe bevorzugt. Die einzelnen Ringe können dabei vollständig unverbunden voneinander angeordnet sein oder bandförmig aufgereiht, beispielsweise durch entsprechende Verwicklungen oder Verschlingungen auch in Form von Knoten, vorliegen.

Die für spiralförmige Umwicklungen beschriebenen Abstände sind auch für die Anbringung von Ringen anwendbar. Ebenso sind die für Bänder beschriebenen Dimensionen auch auf Ringe übertragbar.

Zur vereinfachten Entfernung der Ringe können an den Ringen Sollbruchstellen vorgesehen sein. Ebenso können die Ringe Abzugselemente umfassen, insbesondere laschenartige Anhängsel, die ein einfaches Ergreifen der Ringe ermöglichen. Solche Abzugselemente können auch bereits bei der Anbringung der Ringe hilfreich sein.

Ein Ballon, der mit Restriktionselementen versehen wurde, soll nachfolgend als "präparierter Ballon" bezeichnet werden.

In einer alternativen Ausführungsform kann um den präparierten Ballon in einem weiteren Schritt (B1), also einem Schritt nach Schritt (B) und vor Schritt (C), eine Hülse gestülpt werden, deren Länge vorzugsweise zumindest der Länge des Ballons entspricht. Der Innendurchmesser der Hülse ist vorzugsweise so zu wählen, dass die Innenwandung der Hülse am Ballon reibschlüssig anliegt. Unter einer Hülse ist ein schlauch- oder rohrförmiges Element zu verstehen.

Die Hülse schützt den empfindlichen Ballon beim Durchziehen durch die Öffnung vor Beschädigungen und dient ebenfalls dazu, die Lage der Restriktionselemente zu stabilisieren, also ein Verrutschen oder Verschieben der Restriktionselemente bei der weiteren Handhabung zu verhindern. Das zusätzliche Vorsehen einer solchen Hülse, vorzugsweise einer schlauchförmigen Hülse, ist insbesondere in Verbindung mit der Verwendung von Ringen als Restriktionselementen von Vorteil.

Als Materialien für die Hülse kommen insbesondere Materialien in Frage, die eine geringe Reibung erzeugen, damit das Material des darunter liegenden expandierbaren Elements nicht geschädigt wird. Besonders geeignet ist Polytetrafluorethylen (PTFE, Teflon). Möglich ist jedoch auch die Verwendung anderer Materialien, insbesondere Kunststoffmaterialien, die eine geringe Reibung aufweisen.

Die Öffnung, durch die der präparierte Ballon geführt werden soll, ist so beschaffen, dass sich beim Durchführen des präparierten Ballons der Außendurchmesser des Ballons und ggf. der Hülse reduzieren, d. h. der Innendurchmesser der Öffnung wird vorzugsweise so gewählt, dass er kleiner ist als der Außendurchmesser des präparierten Ballons, andererseits jedoch nicht so viel kleiner, als dass ein Hindurchführen des präparierten Ballons nahezu unmöglich gemacht würde. Entsprechendes gilt für einen mit einer Hülse versehenen präparierten Ballon.

In der Regel ist der Durchmesser der Öffnung so zu wählen, dass ein Hindurchführen des präparierten Ballons nur mit einem gewissen Kraftaufwand möglich ist, der dann zu der gewünschten Durchmesserreduzierung führt.

In der Regel hat die Öffnung einen kreisförmigen Querschnitt. Allgemein ist unter Öffnung im Sinne der Erfindung jegliche Art von Ausnehmung zu verstehen, durch die sich der präparierte Ballon hindurchführen lässt. Beispielsweise kann es sich um eine durchgehende Öffnung innerhalb eines Objekts oder einer Platte handeln, es kann jedoch auch ein Ring sein. Auch eine Öffnung mit einer gewissen Tiefe wie eine Düse wird als Öffnung im Sinne der Erfindung verstanden.

Vorteilhafterweise ist die Öffnung kegelförmig ausgebildet, mit einem größeren ersten Innendurchmesser am ersten Ende, an dem der präparierte Ballon in die Öffnung eingeführt wird, und mit einem kleineren zweiten Innendurchmesser am zweiten Ende, durch das der präparierte Ballon die Öffnung wieder verlässt.

Der erste Innendurchmesser ist so zu wählen, dass er ein einfaches Einführen des präparierten Ballons ggf. mit Hülse ermöglicht. Der zweite Innenmesser ist so zu wählen, dass das Durchziehen des präparierten Ballons ggf. mit Hülse auch tatsächlich zu einer Durchmessereduzierung führt. Anders gesagt ist der erste Innendurchmesser der Öffnung gleich groß oder größer als der Außendurchmesser des präparierten Ballons und der zweite Innendurchmesser der Öffnung ist kleiner als der Außendurchmesser des präparierten Ballons.

Normalerweise werden nach dem Durchführen des präparierten Ballons durch die Öffnung und vor dem Aufbringen der Umhüllung die Restriktionselemente und ggf. die Hülse vom Ballon entfernt (Schritt C1). Die spiralförmige oder ringförmige Riffelstruktur wird dem Ballon bzw. den Falten auf dem Ballon durch die Restriktionselemente und die enge Öffnung aufgeprägt, d. h. sie bleibt auch erhalten, wenn die Restriktionselemente und ggf. die Hülse entfernt werden. Die Aufprägung der beschriebenen Struktur erhöht zugleich die Flexibilität des Ballons, was beim Vorschieben durch englumige Blutgefäße von Vorteil ist.

Denkbar ist aber auch, dass man die Restriktionselemente und ggf. die Hülse auf dem Ballon belässt und die Umhüllung überzieht. In diesem Fall müssen dann vor dem Einsatz des Ballonkatheters nicht nur die Umhüllung, sondern auch die Restriktionselemente und ggf. die Hülse entfernt werden.

Die über den Ballon gezogene Umhüllung dient als Protektor des Ballons und schützt diesen vor Beschädigungen etc. während der Lagerung. Darüber hinaus sorgt die Umhüllung bei mit Wirkstoff beschichteten Ballons dafür, dass dieser auf dem Ballon verbleibt und sich nicht vorzeitig ablöst. Bei der Umhüllung handelt es sich typischerweise um einen schlauchförmigen Überzug.

Bei Bedarf kann der Schritt (C) zur Reduzierung des Ballondurchmessers wiederholt werden, bis die gewünschte Durchmesserreduzierung erreicht ist. Dabei kann jeweils eine Öffnung mit einem verringerten Durchmesser verwendet werden, d. h. bei Wiederholung des Schritts (C) wird der Innendurchmesser der Öffnung jeweils reduziert.

Anstelle oder zusätzlich zur Wiederholung des Schritts (C) ist es auch möglich, die Schritte (B), (C) und (C1) jeweils zu wiederholen, d. h. es wird nicht nur das Durchführen durch eine Öffnung bzw. das Durchführen durch eine Sequenz von Öffnungen wiederholt, sondern auch das Anbringen und Entfernen der Restriktionselemente. Auf diese Weise kann bei erneutem Anbringen beispielsweise eine strammere Wicklung vorgenommen werden oder es können Ringe kleineren Innendurchmessers verwendet werden, um den Außendurchmesser des Ballons noch weiter zu reduzieren.

Ebenso ist in diesem Zusammenhang die Wiederholung des Schrittes (B1) möglich, also das Aufbringen einer Hülse auf dem präparierten Ballon, wobei es sich von selbst versteht, dass die vorherige Hülse in der Regel vor Aufbringung einer neuen Hülse zu entfernen ist.

Der erfindungsgemäße Ballonkatheter weist typischerweise Lumen, vorzugsweise zumindest zwei Lumen auf, wobei ein Lumen der Fluidzufuhr und Druckbeaufschlagung dient und mit dem Balloninneren in Verbindung steht, während das andere Lumen der Aufnahme eines Führungsdrahts dient, der zunächst zum Zielort im Blutgefäß vorgeschoben wird, um anschließend den Ballonkatheter über den Führungsdraht an den Zielort zu bringen. In diesem Zusammenhang sind im Wesentlichen zwei unterschiedliche Systeme aus dem Stand der Technik bekannt, nämlich Over-The-Wire (OTW)- und Rapid Exchange (Rx)-Ballonkatheter. Der erfindungsgemäße Ballonkatheter kann sowohl als OTW- als auch als Rx-Ballonkatheter vorliegen. Während sich bei einem OTW-Katheter das Lumen für den Führungsdraht über die gesamte Länge des Katheters von proximal nach distal erstreckt, verfügt der Rx-Katheter über eine separate Zuführöffnung für den Führungsdraht (Rx-Port), an der der Führungsdraht deutlich distal des proximalen Endes des Katheters aus dem Katheter austritt. Entsprechend laufen die Lumen für die Fluidzufuhr und den Führungsdraht im Falle eines OTW-Ballonkatheters konzentrisch oder parallel zueinander vom proximalen Ende des Katheters bis zum Ballon, während dies bei einem Rx-Katheter nur zwischen Rx-Port und Ballon der Fall ist. Der Abschnitt zwischen Rx-Port und proximalem Ende hingegen weist nur ein Lumen für die Fluidzufuhr auf. Typischerweise verlaufen die Lumen in den Bereichen, in denen der Katheter zwei Lumen aufweist, parallel zueinander, wobei auch ein inneres Lumen durch das weitere äußere Lumen verlaufen kann.

Während der Durchführung des Schritts (C), d. h. der Reduzierung des Ballondurchmessers mittels Durchführen des präparierten Ballons durch eine Öffnung, erstreckt sich der Führungsdraht vorzugsweise durch das hierfür vorgesehene Lumen des Ballonkatheters. Auf diese Weise wird verhindert, dass das Lumen für den Führungsdraht komprimiert und verengt wird. Für den späteren Einsatz als Ballonkatheter ist es wichtig, dass der Führungsdraht gegenüber dem Ballonkatheter durch das entsprechende Lumen problemlos in Längsrichtung nach distal oder proximal bewegt werden kann, weshalb eine Verengung des Lumens nachteilig ist. Dies gilt unabhängig davon, ob es sich um einen OTW- oder einen Rx-Katheter handelt, denn im Rahmen des Schritts (C) wird eine radial nach innen wirkende Kraft in erster Linie im Ballonbereich des Ballonkatheters ausgeübt, da dieser einen erheblich größeren Querschnitt als Bereiche des Katheters ohne Ballon aufweist. Sowohl der OTW- als auch der Rx-Katheter verfügen aber im Bereich des Ballons über ein Führungsdrahtlumen, dessen Verengung vermieden werden sollte.

Vorzugsweise verläuft der Führungsdraht bereits während des Schritts (B) und ggf. (B1) durch das Führungsdrahtlumen des Ballons. Auf diese Weise wird sichergestellt, dass auch beim engen Anlegen und Strammziehen der Restriktionselemente eine Verengung des Führungsdrahtlumens vermieden wird.

Das Material der Restriktionselemente sollte so gewählt werden, dass dieses an keinem Teil des Katheters haftet. Diese Anforderung eines nicht haftenden Restriktionselements wird insbesondere durch Polytetrafluorethylen (PTFE)-haltige Materialien erfüllt.

Sofern eine mehrlagige Anbringung vorgesehen ist, genügt es in der Regel, wenn die direkt auf dem Ballon aufliegenden Restriktionselemente nicht haftende Eigenschaften aufweisen, also beispielsweise aus PTFE-haltigen Materialien bestehen.

Besondere Bedeutung hat die Erfindung für mit Wirkstoff beladene Ballons, auch Drug Eluting Balloons (DEB) genannt. Diese weisen meist einen größeren Querschnitt auf als unbeladene Ballons, z. B. weil sich zwischen den Falten, in die der Ballon im kontrahierten Zustand gelegt wird, ein Wirkstoff wie Paclitaxel befindet. Insbesondere bei sehr langen Ballons macht dies das Vorschieben im Blutgefäßsystem schwierig. Bevorzugt sind die Wirkstoffe ausgewählt aus der Gruppe: Tretinoin, Orphanrezeptoragonisten, Elafinderivate, Corticosteroide, Steroidhormone, Paclitaxel, Rapamycin, Tacrolimus, hydrophobe Proteine und/oder zellproliferationsverändernde Substanzen.

Neben dem erfindungsgemäßen Verfahren betrifft die Erfindung auch einen Ballonkatheter, der nach dem erfindungsgemäßen Verfahren erhalten wird.

Ballonkatheter sind grundsätzlich hinlänglich im Stand der Technik bekannt und weisen einen langgestreckten, von proximal nach distal verlaufenden Katheterschaft sowie einen im distalen Bereich angeordneten Ballon auf. Es handelt sich um einen Katheter, der hinsichtlich seiner Dimensionen auf die Einführung in ein Körperlumen, insbesondere ein (Blut)gefäßsystem, abgestimmt ist. Dabei können die exakten Dimensionen variieren, je nachdem, ob das Blutgefäß bspw. eine Koronararterie, ein intrakranielles Blutgefäß oder eine Unterschenkelarterie ist. Darüber hinaus verfügt der Ballonkatheter über Mittel zur Zuführung eines Fluids zum Ballon. Hierbei kann es sich um ein Zufuhrlumen handeln, das sich über die Länge des Ballonkatheters erstreckt.

Des Weiteren kann der erfindungsgemäße Ballonkatheter nicht nur der Beseitigung von Stenosen und der lokalen Wirkstoffeinbringung, sondern zusätzlich der Platzierung eines Stents (Endoprothese) im Körperlumen dienen. Stents sind röhrenartige Stützstrukturen, die in einem Körperlumen, bspw. einem Blutgefäß implantiert werden, um dieses dauerhaft offen zu halten. Derartige Stents können selbstexpandierend sein oder mit Hilfe eines Ballons expandiert werden. Hierzu wird der Stent auf dem Ballon aufgecrimpt und mit Hilfe des Ballonkatheters in das Körperlumen eingebracht. An der vorgesehenen Stelle wird sodann der Ballon durch Zufuhr eines Fluids expandiert, wodurch sich auch der Stent weitet und im Körperlumen verankert wird. Schließlich wird der Ballon wieder zusammengezogen und aus dem Körperlumen entfernt, während der Stent im Körperlumen verbleibt.

Am proximalen Ende des Ballonkatheters ist zumeist ein sog. Katheterhub vorgesehen, d. h. ein Anschlussstück für die Vorrichtung zur Fluidzufuhr und Druckbeaufschlagung. Die Verbindung kann z. B. eine herkömmliche Luer- bzw. Luer-Lock-Verbindung sein. Unter proximal wird in Richtung Körperäußeres, d. h. zum behandelnden Arzt hin verstanden, unter distal die entgegengesetzte Richtung, d. h. in Richtung des zu behandelnden Blutgefäßes. Die Einfuhr des Ballonkatheters in den menschlichen Körper erfolgt zumeist in der Leistengegend über die Arteria femoralis.

Entlang des Ballonkatheters können an verschiedenen Positionen röntgendichte Markierungen angebracht sein, die der Visualisierung des Katheters im Röntgenbild dienen. Insbesondere kann es sich dabei um Markierungen aus Platin oder einer Platinlegierung handeln.

Das erfindungsgemäße Verfahren weist gegenüber dem Stand der Technik den Vorteil auf, dass die mechanischen Belastungen des Ballons, die ursächlich für mögliche Beschädigungen desselben sind, auf ein Minimum reduziert werden. Dies liegt vor allem an der Anbringung der erfindungsgemäßen Restriktionselemente direkt auf dem Ballon durch Umwickeln oder Aufschieben. Im Gegensatz zu anderen Verfahren treten hierbei wesentlich geringere Zugkräfte als bei bekannten Verfahren aus dem Stand der Technik auf.

Sämtliche Ausführungen, die zum Herstellungsverfahren gemacht wurden, gelten in entsprechender Weise für den Ballon sowie den Ballonkatheter und umgekehrt.

Die Erfindung wird anhand der Figuren beispielhaft näher erläutert. Es ist darauf hinzuweisen, dass die Figuren bevorzugte Ausführungsvarianten der Erfindung zeigen, die Erfindung ist jedoch nicht darauf beschränkt.

Es zeigen:
- Fig. 1: Einen Ballon eines Ballonkatheters mit bandförmigen Restriktionselementen;
- Fig.2: den Ballon gemäß Fig. 1 nach Strammziehen der bandförmigen Restriktionselemente;
- Fig. 3: den Ballon gemäß Fig. 2 nach dem Durchführen durch eine Öffnung;
- Fig. 4: einen Ballon eines Ballonkatheters mit ringförmigen Restriktionselementen;
- Fig. 5: den Ballon gemäß Fig. 4 nach Anziehen der ringförmigen Restriktionselemente und
- Fig. 6: den Ballon gemäß Fig. 5 nach dem Durchführen durch eine Öffnung.

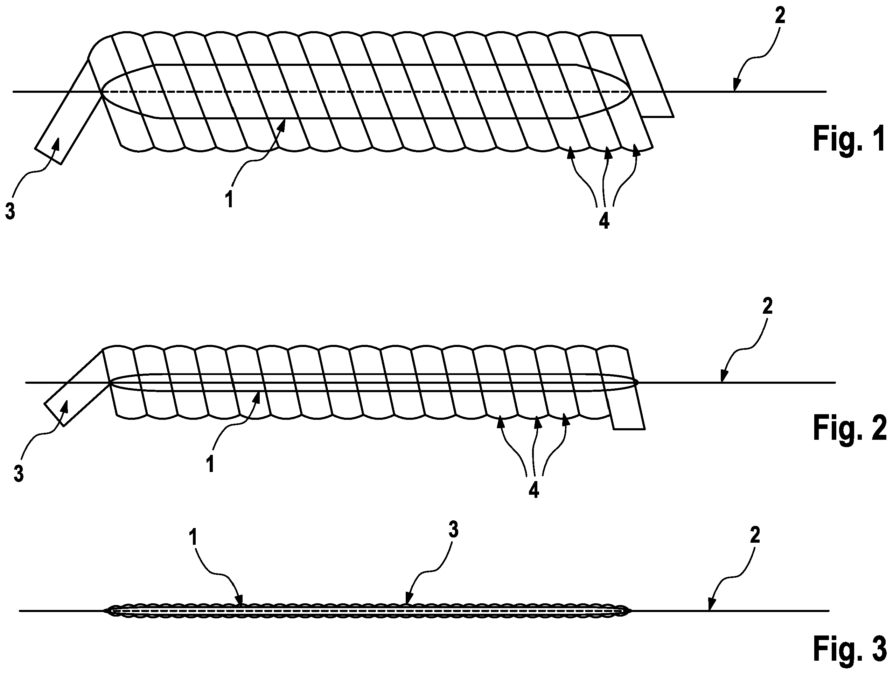

Figur 1 zeigt einen Teil eines Ballonkatheters, nämlich den Katheterschaft 2 mit dem am distalen Ende angeordneten Ballon 1. Um den Ballon 1 ist als Restriktionselement ein Band 3 vorzugsweise aus PTFE gelegt, sodass sich eine Vielzahl von Windungen 4 ergibt.

Figur 2 zeigt den Ballon 1 aus Fig. 1, jedoch nachdem das um den Ballon 1 gelegte bandförmige Restriktionselement 3 und die Windungen 4 strammgezogen wurden. Bereits dies bewirkt eine signifikante Reduzierung des Außendurchmessers des Ballons 1, die jedoch noch nicht ausreichend ist.

Figur 3 zeigt das Ergebnis nach dem Durchführen des Ballons 1 mit einem in Windungen 4 gelegten bandförmigen Restriktionselement 3 durch eine hier nicht dargestellte Öffnung. Der Außendurchmesser des Ballons 1 wurde erheblich reduziert. Anschließend kann das bandförmige Restriktionselement 3 entfernt und der Ballon 1 mit einer Umhüllung als Protektor versehen werden.

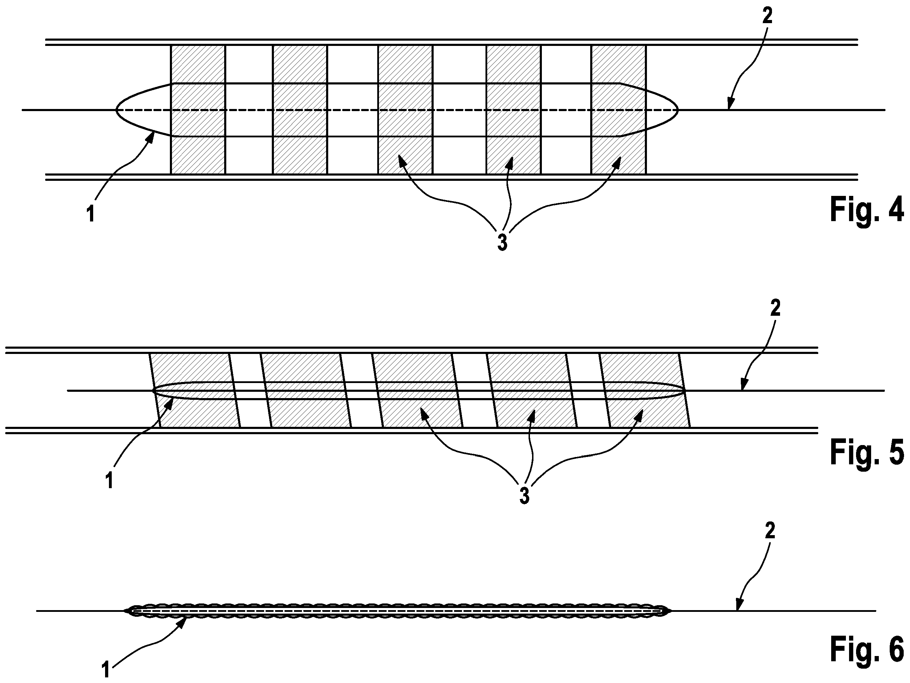

Figur 4 zeigt einen Teil eines Ballonkatheters, nämlich den Katheterschaft 2 mit dem am distalen Ende angeordneten Ballon 1. Um den Ballon 1 ist eine Vielzahl ringförmiger Restriktionselemente 3 vorzugsweise aus PTFE gelegt.

Figur 5 zeigt den Ballon 1 aus Fig. 4, jedoch nachdem sich die um den Ballon 1 gelegten ringförmigen Restriktionselemente 3 zusammengezogen und entsprechend leicht verbreitert haben. Bereits dies bewirkt eine signifikante Reduzierung des Außendurchmessers des Ballons 1, die jedoch noch nicht ausreichend ist.

Figur 6 zeigt das Ergebnis nach dem Durchführen des Ballons 1 mit den ringförmigen Restriktionselementen 3 durch eine hier nicht dargestellte Öffnung. Der Außendurchmesser des Ballons 1 wurde erheblich reduziert. Anschließend können die ringförmigen Restriktionselemente 3 entfernt und der Ballon 1 mit einer Umhüllung als Protektor versehen werden.

## Patentansprüche

1. Verfahren zur Reduzierung des Außendurchmessers des Ballons (1) eines Ballonkatheters im nicht expandierten Zustand, umfassend folgende Schritte:
(A) Bereitstellen eines Ballonkatheters;
(B) Anbringen von zumindest einem Restriktionselement (3) auf zumindest einem Teil des Ballons (1) des Ballonkatheters, wobei das zumindest eine Restriktionselement (3) als Band oder Ring vorgesehen ist;
(C) Durchführen des gemäß Schritt (B) präparierten Ballons (1) durch eine Öffnung; und
(D) Aufbringen einer Umhüllung auf den Ballon (1).

2. Verfahren nach Anspruch 1, **dadurch gekennzeichnet, dass** die Restriktionselemente (3) in einem zusätzlichen Schritt (C1), nämlich nach Schritt (C) und vor Schritt (D), von dem Ballon (1) entfernt werden.

3. Verfahren nach Anspruch 1 oder 2, **dadurch gekennzeichnet, dass** der Ballon (1) vor dem Anbringen der Restriktionselemente (3) gemäß Schritt (B) in Falten gelegt wird.

4. Verfahren nach einem der Ansprüche 1 bis 3, **dadurch gekennzeichnet, dass** Schritt (C) wiederholt wird, bis der gewünschte Außendurchmesser des Ballons (1) erreicht ist.

5. Verfahren nach einem der Ansprüche 1 bis 4, **dadurch gekennzeichnet, dass** über den präparierten Ballon in einem Schritt (B1) eine Hülse geschoben wird, deren Länge zumindest der Länge des Ballons (1) entspricht, wobei Schritt (B1) nach Schritt (B), aber vor Schritt (C) durchgeführt wird.

6. Verfahren nach Anspruch 2, bevorzugt in Kombination mit einem der Ansprüche 3 bis 5, **dadurch gekennzeichnet, dass** die Schritte (B) bis (C1) wiederholt werden, bis der gewünschte Außendurchmesser des Ballons (1) erreicht ist.

7. Verfahren nach einem der Ansprüche 1 bis 6, **dadurch gekennzeichnet, dass** der Ballonkatheter ein Lumen für einen Führungsdraht aufweist, das sich zumindest durch den Ballon (1) erstreckt, wobei während des Schritts (C), bevorzugt während der Schritte (B) und (C) sowie ggf. (B1) der Führungsdraht durch das Lumen verläuft.

8. Verfahren nach einem der Ansprüche 1 bis 7, **dadurch gekennzeichnet, dass** die Restriktionselemente (3) ganz oder teilweise aus Polytetrafluorethylen gefertigt sind.

9. Verfahren nach einem der Ansprüche 1 bis 8, **dadurch gekennzeichnet, dass** die Restriktionselemente (3) eine Breite ≥ 2 mm, vorzugsweise von 0,5 bis 2 cm aufweisen.

10. Verfahren nach einem der Ansprüche 1 bis 9, **dadurch gekennzeichnet, dass** der Abstand zwischen den einzelnen Windungen des Bandes bzw. den einzelnen Ringen (3) 0,5 bis 7 mm, bevorzugt 1 bis 5 mm beträgt.

11. Verfahren nach einem der Ansprüche 1 bis 10, **dadurch gekennzeichnet, dass** als Restriktionselemente (3) mehrere Bänder und/oder Ringe vorgesehen sind.

12. Verfahren nach Anspruch 11, **dadurch gekennzeichnet, dass** die Anbringung der Bänder spiralförmig, schraubenförmig, helikal oder ringförmig vorgenommen wird.

13. Verfahren nach einem der Ansprüche 1 bis 12, **dadurch gekennzeichnet, dass** die Öffnung kegelförmig ausgebildet ist mit einem größeren ersten Innendurchmesser am ersten Ende, an dem der präparierte Ballon (1) in die Öffnung eingeführt wird, und mit einem kleineren zweiten Innendurchmesser am zweiten Ende, durch das der präparierte Ballon (1) die Öffnung wieder verlässt.

## Claims

1. Method for reducing the outer diameter of the balloon (1) of a balloon catheter in the non-expanded state, comprising the following steps:
(A) Provision of a balloon catheter;
(B) Attaching at least one restriction element (3) to at least a portion of the balloon (1) of the balloon catheter, wherein the at least one restriction element (3) is provided as band or ring;
(C) Passing the balloon (1) prepared in accordance with step (B) through an opening; and
(D) Applying a sheath to the balloon (1).

2. Method according to claim 1, **characterized in that** the restriction elements (3) are removed from the balloon (1) in an additional step (C1), namely after step (C) and before step (D).

3. Method according to claim 1 or 2, **characterized in that** the balloon (1) is provided with folds before applying the restriction elements (3) pursuant to step (B).

4. Method according to any one of claims 1 to 3, **characterized in that** step (C) is repeated until the desired outer diameter of the balloon (1) is achieved.

5. Method according to any one of claims 1 to 4, **characterized in that** in a step (B1) a sleeve, the length of which amounts to at least the length of the balloon (1), is slid over the prepared balloon, said step (B1) being carried out after step (B) but before step (C).

6. Method according to claim 2, preferred in combination with one of the claims 3 to 5, **characterized in that** the steps (B) to (C1) are repeated until the desired outer diameter of the balloon (1) is achieved.

7. Method according to any one of claims 1 to 6, **characterized in that** the balloon catheter is provided with a lumen for a guidewire extending at least through the balloon (1), wherein during step (C), preferably during steps (B) and (C) as well as (B1), if applicable, the guidewire passes through the lumen.

8. Method according to any one of claims 1 to 7, **characterized in that** the restriction elements (3) are made wholly or in part of polytetrafluoroethylene.

9. Method according to any one of claims 1 to 8, **characterized in that** the restriction elements (3) have a width of ≥ 2 mm, preferably ranging between 0.5 and 2 cm.

10. Method according to any one of claims 1 to 9, **characterized in that** the distance between the individual turns/windings of the band or the individual rings (3) ranges between 0.5 and 7 mm, preferably between 1 and 5 mm.

11. Method according to any one of claims 1 to 10, **characterized in that** several bands and/or rings are provided as restriction elements (3).

12. Method according to claim 11, **characterized in that** the attachment/arrangement of the bands is carried out spirally, screw-like, helically or in the form of rings.

13. Method according to any one of claims 1 to 12, **characterized in that** the opening has a tapered configuration, with a larger first inner diameter at the first end where the prepared balloon (1) is introduced into the opening, and with a smaller second inner diameter at the second end through which the prepared balloon (1) exits the opening.

## Revendications

1. Procédé permettant de réduire le diamètre extérieur du ballon (1) d'un cathéter à ballonnet à l'état non dilaté, comprenant les étapes suivantes:
(A) Fournir un cathéter à ballonnet;
(B) Mise en place d'au moins un élément de restriction (3) sur au moins une partie du ballon (1) du cathéter à ballon, ledit au moins un élément de restriction (3) étant réalisé sous la forme d'une bande ou d'un anneau;
(C) Faire passer le ballon (1) préparé conformément à l'étape (B) à travers une ouverture; et
(D) Appliquer une enveloppe sur le ballon (1).

2. Procédé selon la revendication 1, **caractérisé en ce que** les éléments de restriction (3) sont retirés du ballon (1) au cours d'une étape supplémentaire (C1), à savoir après l'étape (C) et avant l'étape (D).

3. Procédé selon la revendication 1 ou 2, **caractérisé en ce que** le ballon (1) est plissé avant la mise en place des éléments de restriction (3) conformément à l'étape (B).

4. Procédé selon l'une des revendications 1 à 3, **caractérisé en ce que** l'étape (C) est répétée jusqu'à ce que le diamètre extérieur souhaité du ballon soit atteint.

5. Procédé selon l'une des revendications 1 à 4, **caractérisé en ce qu'**un manchon, dont la longueur correspond au moins à la longueur du ballon (1), est enfilé sur le ballon préparé au cours d'une étape (B1), l'étape (B1) étant réalisée après l'étape (B), mais avant l'étape (C).

6. Procédé selon la revendication 2, de préférence en combinaison avec l'une des revendications 3 à 5, **caractérisé en ce que** les étapes (B) à (C1) sont répétées jusqu'à ce que le diamètre extérieur souhaité du ballon (1) soit atteint.

7. Procédé selon l'une des revendications 1 à 6, **caractérisé en ce que** le cathéter à ballonnet comporte une lumière pour un fil-guide qui s'étend au moins à travers le ballonnet (1), le fil-guide passant à travers la lumière pendant l'étape (C), de préférence pendant les étapes (B) et (C) ainsi que, le cas échéant, (B1).

8. Procédé selon l'une des revendications 1 à 7, **caractérisé en ce que** les éléments de restriction (3) sont entièrement ou partiellement fabriqués en polytétrafluoroéthylène.

9. Procédé selon l'une des revendications 1 à 8, **caractérisé en ce que** les éléments de restriction (3) présentent une largeur ≥ 2 mm, de préférence comprise entre 0,5 et 2 cm.

10. Procédé selon l'une des revendications 1 à 9, **caractérisé en ce que** la distance entre les différentes spires de la bande ou les différents anneaux (3) est comprise entre 0,5 et 7 mm, de préférence entre 1 et 5 mm.

11. Procédé selon l'une des revendications 1 à 10, **caractérisé en ce que** plusieurs bandes et/ou anneaux sont prévus comme éléments de restriction (3).

12. Procédé selon la revendication 11, **caractérisé en ce que** la mise en place des bandes s'effectue en spirale, en hélice ou en anneau.

13. Procédé selon l'une des revendications 1 à 12, **caractérisé en ce que** l'ouverture est de forme conique, avec un premier diamètre intérieur plus grand à la première extrémité, par laquelle le ballon préparé (1) est introduit dans l'ouverture, et avec un deuxième diamètre intérieur plus petit à la deuxième extrémité, par laquelle le ballon préparé (1) quitte à nouveau l'ouverture.
